# EUROPEAN PATENT APPLICATION

(11) **EP 4 257 669 A1**
(43) Date of publication of application: **11.10.2023**
(21) Application number: 22167187.8
(22) Date of filing: 07.04.2022
(51) Int. Cl.: C12M 3/00, B01L 9/02, C12M 1/36

(54) **MODULAR SYSTEM FOR PROVIDING A BIOPROCESS DEVICE ASSEMBLY**

(71) Applicant: Sartorius Stedim Biotech GmbH, 37079 Göttingen (DE)
(72) Inventor: Scholz, Jochen, 37079 Göttingen (DE); Grimm, Christian, 37079 Göttingen (DE)
(74) Representative: Prinz & Partner mbB

(57) **Abstract**

A modular system for providing a bioprocess device assembly comprises a rigid skid (10) and a plurality of grid-modules (12). The skid (10) includes a plurality of identical plug-in structures (14) being arranged in a regular two-dimensional grid. The grid arrangement of the plug-in structures (14) defines two-dimensional plug-in fields (20) of an identical standard shape and size. At least some of the grid-modules (12) have a matching counterpart plug-in structure (22) adapted to fix the respective grid-module (12) to any of the plug-in structures (14) of the skid (10). At least some of the grid-modules (12), in an installed state when fixed to a plug-in structure (14) of the skid (10) and/or to a neighboring grid-module (12), have a two-dimensional extension in the plane of the grid that is not greater than the standard size of the plug-in fields (20). At least some of the grid-modules (12) include at least one connection port (30) adapted to receive a rigid universal flow connector (26; 34) of standard shape and size in a standard position and orientation. The connection port (30) of each grid-module (12) defines the standard position and orientation of the universal flow connector (26; 34) such that, in its installed state, the grid-module (12) can be connected via the universal flow connector (26; 34) either directly to the connection port (30) or to another universal flow connector (34) received in the connection port (30) of an opposing grid-module (12) fixed to the grid-module (12) and/or to a neighboring plug-in structure (14). At least some of the grid-modules (12) have at least one integrated fluid line (32) through, or into, which a medium to be processed or analyzed can flow. The integrated fluid line (32) is in flow communication with the connection port (30) of the grid-module (12). The plurality of grid-modules (12) includes at least one flow control grid-module having means for actively changing flow properties of the medium, and/or at least one interaction grid-module having means for sensing, detecting or measuring a property of the medium.

## Description

The invention relates to a modular system for providing a bioprocess device assembly. The invention further relates to a computer program including an algorithm for creating a digital simulation of a bioprocess device assembly configured by a user based on such a modular system.

In general, processes in which single-use devices are used are becoming increasingly widespread in the pharmaceutical production of high-quality active substances due to the high flexibility that can be achieved and the savings in time, investment, and operating expenses such as cleaning and inspection. Furthermore, it is becoming increasingly important for the developers and manufacturers of biopharmaceutical products to be able to quickly adapt manufacturing processes.

Prefabricated standardized device arrangements which can be configured in many ways are often used to perform specific unit operations. One example of such a solution is the FlexAct^{®} System from Sartorius Stedim Biotech GmbH, which is a work platform that links together a variety of biomanufacturing tasks. The FlexAct^{®} System is flexibly adaptable and can be used, among other things, for buffer preparation, cell collection, virus inactivation, media preparation, virus depletion, line testing or bag testing. The core of this device arrangement is a compact central multifunctional operating module in the form of a stainless-steel trolley having a control unit and an operating panel. With the FlexAct^{®} System specific unit operations using selected single-use process components (bags, hose lines, connectors, filter capsules, etc.) can be performed in an at least partially automated manner.

The device arrangements known from the prior art are subject to certain restrictions. Usually, a device arrangement is designed for a specific process step with a specific volume. Installation or conversion of the device arrangement on the part of the user can only be carried out with great effort, which leads to an increased susceptibility to errors. In this context, in particular the fast and correct connection of single-use process components is a challenge. Furthermore, it is necessary to perform an adjustment between the basic components of the device arrangement (e.g., a pump) with the single-use process components connected thereto and further used single-use process components ("wetware"), and the control software (selected process step, basic conditions).

WO 2019/185356 A1 shows a configurable device for the flexible provision of connections and/or functions in a biopharmaceutical process. The configurable device includes a body in which predefined pipe sections and plug-in locations are formed by recesses in the material of the body. The configurable device further includes a plurality of functional elements which are adapted to be inserted into the plug-in locations.

From WO 2020/099382 A1 another type of a configurable device arrangement for performing at least one unit operation in a biopharmaceutical process is known The device arrangement includes a base rack and a plurality of holders for releasable direct or indirect attachment of process components, in particular single-use process components, for the unit operation. The holders are in turn adapted to be releasably attached directly or indirectly to the base rack. The device arrangement further includes a positioning system which defines specific positions for the holders relative to the base rack.

WO 2022/012981 A1 discloses a device assembly for producing bioconjugates, comprising a conjugation unit for performing a bioconjugation reaction in a medium, a first filtration unit for separating precipitates and/or agglomerates, and a second filtration unit for performing an ultrafiltration and/or a diafiltration process. The first filtration unit is arranged in a flow path between the conjugation unit and the second filtration unit. The second filtration unit comprises a single-use loop-assembly for recirculating the medium in the ultrafiltration and/or diafiltration process which can be mounted to and/or dismounted from a basic structure of the device assembly as a whole, preferably together with a single-use conjugation bag and a single-use recirculation bag and other single-use components of the device assembly. The device assembly further comprises a single control unit for controlling the transfer of medium from the conjugation unit through the first filtration unit to the second filtration unit and for controlling the ultrafiltration and/or diafiltration process.

It is an object of the invention to overcome the limitations of the device arrangements known from the prior art and to make it possible to easily provide individually configured bioprocess device assemblies with great flexibility.

The above problem is solved by a modular system according to claim 1. Advantageous and expedient embodiments of the invention are apparent from the dependent claims.

The invention provides a modular system for providing a bioprocess device assembly and comprises a rigid skid and a plurality of grid-modules. The skid includes a plurality of identical plug-in structures being arranged in a regular two-dimensional grid. The grid arrangement of the plug-in structures defines two-dimensional plug-in fields of an identical standard shape and size. At least some of the grid-modules have a matching counterpart plug-in structure adapted to fix the respective grid-module to any of the plug-in structures of the skid. At least some of the grid-modules, in an installed state when fixed to a plug-in structure of the skid and/or to a neighboring grid-module, have a two-dimensional extension in the plane of the grid that is not greater than the standard size of the plug-in fields. At least some of the grid-modules include at least one connection port adapted to receive a rigid universal flow connector of standard shape and size in a standard position and orientation. The connection port of each grid-module defines the standard position and orientation of the universal flow connector such that, in its installed state, the grid-module can be connected via the universal flow connector directly to the connection port of an opposing grid-module fixed to the grid-module or to a neighboring plug-in structure. As an alternative, the grid-module can be connected via the universal flow connector to another universal flow connector received in the connection port of an opposing grid-module fixed to a neighboring plug-in structure. At least some of the grid-modules have at least one integrated fluid line through, or into, which a medium to be processed or analyzed can flow. The integrated fluid line is in flow communication with the connection port of the grid-module. The plurality of grid-modules includes at least one flow control grid-module having means for actively changing flow properties of the medium, and/or at least one interaction grid-module having means for sensing, detecting or measuring a property of the medium.

The modular system allows a plurality of interacting grid-modules to be detachably fixed in any of a plurality of defined positions on a skid to create individually configured bioprocess device assemblies. Due to the direct flow connection between neighboring grid-modules, compact tubeless setups can be realized. A great flexibility is achieved because the grid-modules can be replaced or exchanged easily. The invention allows to create a flexible trial kit in process development on the one hand, and a fully self-contained, preassembled GMP (good manufacturing practice) ready production assembly on the other hand.

In the given context, a rigid skid is to be understood as a stiff frame, plate or any other support structure providing a plurality of regularly arranged plug-in structures, preferably on a generally flat surface, for carrying a plurality of grid-modules. The skid can be part of a work platform or a control tower or a similar system for assisting the performance of unit operations.

According to the invention, the plug-in structures of the skid are arranged in a regular two-dimensional grid. This means that the plug-in fields defined by the positions of the regularly arranged plug-in structures are all of identical shape and size. In particular, the shape is that of a rectangle (e.g. a square) or a regular polygon (equiangular and equilateral), resulting in a regular tessellation of the skid. For example, the regular two-dimensional grid can be a rectilinear cartesian grid or a honeycomb grid.

The grid-modules are individual units providing functions that are typically required in a bioprocess device assembly. The various types of grid-modules can be assigned to different groups or categories. According to the invention, the plurality of grid-modules includes at least one flow control grid-module having means for actively changing flow properties of the medium, and/or at least one interaction grid-module having means for sensing, detecting or measuring a property of the medium. A control grid-module can include a valve or a pump, for example, whereas an interaction grid-module can include a sensor or a probe head, for example. Of course, other types of grid-modules can be employed as well, as will be explained later.

The grid-modules typically have a counterpart plug-in structure matching the plug-in structures of the skid. However, it is to be noted that not all of the grid-modules need to have such a counterpart plug-in structure. Some of the grid-modules may be held by one or more of their neighboring grid-modules which, in turn, are fixed to the skid via their counterpart plug-in structures.

Further, some of the grid-modules may have a two-dimensional extension that is greater than the standard size of a regular plug-in field. In particular, the dimension of a grid-module in one of the directions in the plane of the grid can be greater than the corresponding dimension(s) of the standard size plug-in field. However, according to the invention, the dimensions of such a larger grid-module are such that it is ensured that the larger grid-module could be connected to a neighboring grid-module of standard size via a universal flow connector. In simple terms, the dimension of a larger grid-module can be an integer multiple of the corresponding dimension of the plug-in field.

Fluid connection between any neighboring grid-modules having a fluid-related function is provided via universal flow connectors having a standard shape and a standard size. This means that the connectors are not specific to the respective grid-module (type), but common to all grid-modules. Since all grid-modules that need fluid connection are adapted to receive such a universal flow connector in a standard position and orientation, only one type of flow connectors needs to be provided for the whole modular system. According to the invention, the connection port of each grid-module defines the standard position and orientation (relative to the plane of the grid) of the universal flow connector such that, in its installed state, the respective grid-module can be connected via the universal flow connector to the connection port or to a universal flow connector of an opposing grid-module fixed to a neighboring plug-in structure, irrespective of the actual type of the grid-module (as long as it has a fluid connection).

It is to be noted that the universal flow connector can be permanently fixed to or be as an integral part of the grid-module. In such a case, the connection port and the universal flow connector are formed as one piece. From a logical point of view, the connection port is then to be understood as the structure directly surrounding the universal flow connector and supplementing the flow path into or out of the integrated fluid line of the grid-module.

The connection port of a grid-module is in flow communication with an integrated fluid line. For some applications it is sufficient that this fluid line is a dead-end fluid line into which a medium to be analyzed can flow. Such a dead-end can be used as a common measuring location for a number of different parameters, so that only one dead-end fluid line is necessary to measure the various parameters.

The actual shape and size of the grid-modules and the corresponding standard shape and size of the two-dimensional plug-in fields on the skid can be chosen as appropriate. For experimental setups (laboratory scale) for low volumes the grid-modules and the plug-in fields can be smaller as compared to grid-modules used in high-volume commercial manufacturing assemblies (production scale). The same is true for the diameter of the connection ports.

Apart from the flow control and interaction grid-modules, the modular system according to the invention can also comprise other types of grid-modules. In particular, the plurality of grid-modules can further include at least one connector grid-module or at least one manifold grid-module. A connector grid-module provides a simple fluid path for connecting remote grid-modules, whereas a manifold grid-module distributes fluid to several grid-modules or collects fluid from several grid-modules. For example, the fluid lines in a manifold grid-module can have a Y or T or an X configuration.

The modular concept of the invention is most effective in practice with grid-modules whose maximum extensions in the plane of the grid are equal to the corresponding extensions of the plug-in fields. Irrespective of the actual shape of the plug-in fields (e.q. squares or regular hexagons), this allows the greatest flexibility for setting up clear individual configurations which can be easily assembled and changed, if necessary, by the user.

According to a preferred embodiment of the invention, at least one of the grid-modules includes four connection ports in a right-angle arrangement. This allows maximum flexibility since such a grid-module can establish flow connections in all orthogonal directions in the plane of the grid.

The arrangement of grid-modules can even be expanded in a further direction, thus allowing even more complex three-dimensional configurations. To this end, at least one of the grid-modules includes a further plug-in structure allowing another grid-module to be connected in a direction perpendicular to the plane of the grid. According to this concept, grid-modules can be stacked on one another to overcome the limitations of two-dimensional grid-module patterns.

In order to provide a flow connection between stacked grid-modules, at least one grid-module includes a universal flow connector that, in the installed state of the grid-module, is oriented in a direction perpendicular to the plane of the grid.

Apart from flow connections, it is useful to also standardize any electrical and/or optical connections that are necessary in the final bioprocess device assembly. Therefore, at least some of the grid-modules can include at least one universal electrical and/or optical connector adapted to be connected to a universal electrical and/or optical connector of a neighboring grid-module or to an electrical and/or optical interface of a neighboring grid-module.

According to a further development of the invention, the modular system is not limited to fluid-related grid-modules, but further comprises at least one non-fluid grid-module without any fluid connection to a neighboring grid-module, but having at least one universal mechanical and/or electrical and/or optical connector adapted to be connected to a universal mechanical and/or electrical and/or optical connector of a neighboring grid-module.

Some of the grid-modules that are typically used in certain arrangements can be preassembled to form a structural unit before they are fixed to the skid.

In particular, the preassembled group of grid-modules can be held together in an installation box or by an installation frame for easier shipment and installation. According to a variant, the box or frame itself includes flow and/or electrical and/or optical connectors.

The installation box or installation frame itself can also be a skid in the sense of the invention. This means that the installation box or installation frame functions as a support structure and provides a number of regularly arranged plug-in structures for carrying a corresponding number of grid-modules. In this case the installation box or installation frame and the grid-modules fixed therein can constitute an independent (autarchic) bioprocess device assembly in the sense of the invention.

To help the user in setting up a configuration with the modular system and to avoid confusion, at least some of the grid-modules can be provided with a schematic graphical representation of a function associated to the respective grid-module on an outer surface thereof. This allows the user to easily identify the function of a grid-module at any time.

Schematic graphical representations can also be provided in at least some of the plug-in fields of the skid. Especially when certain plug-in fields are reserved for specific grid-modules, such schematic graphical representations of the functions associated to those grid-modules show the user where to place them. According to a further development of the invention, schematic graphical representations can be visualized in the plug-in fields according to a predefined (part of a) configuration scheme or according to a digital configuration scheme created with a computer program. While permanent graphical representations can be realized as printings, etched structures etc., variable graphical representations would require the skid to include one or more displays or illumination means, or the graphical representations are projected on the skid by a projector, or the graphical representations are presented to the user via AR (augmented reality).

To avoid the hassle of cables related to power supply and data/signal transfer, the grid-modules can include a wireless communication module and/or a wireless power receiver.

A major aspect of the invention is the integration of functional components, especially sensors, in some of the grid-modules. To facilitate placing and mounting such functional components, universal receptacles can be used which are adapted to receive universal functional components. "Universal" means that different types of functional components have a design matching a standard design of a receptacle, so that the receptacle is capable of receiving different types of functional components.

Preferably, the universal receptacle is integrated into a fluid line or into a flow connector of the grid-module. It is to be understood that the fluid line or the flow connector itself can be designed such that no additional part is required to receive a functional component, i.e. the fluid line or the flow connector itself can be the universal receptacle.

As already indicated before, at least one of the grid-modules can include several functional components. For example, a flow control grid-module including a valve can further include a sensor or a flow cell, or a grid-module including crossing fluid lines can further include several sensors accommodated in some of the fluid line portions.

As mentioned in the beginning, especially in the pharmaceutical production of high-quality active ingredients single-use equipment is used to an increasing extent due to the high flexibility achievable therewith as well as the saving of time, investments and operating expenditure such as the cleaning, validation and examination of such equipment. Single use equipment is not only used in the field of product and process development anymore, but also in the field of clinical trial manufacturing (CTM) for the approval procedure and even in the commercial good manufacturing practice (GMP) in the production of drugs. Therefore, it is preferred that the grid-modules of the modular system according to the invention are single-use grid-modules made from materials whose properties are not significantly deteriorated when sterilized by ionizing radiation, such as gamma rays or beta rays or X-rays, or by chemicals or by steam. This means that basic characteristics of such materials, like mechanical stability, toughness (opposite of brittleness) etc. and the dimensions of components made from such materials, are not significantly affected by a sterilization process.

According to a preferred embodiment of the invention, the materials used for the grid-modules can be further distinguished as follows: Any components that come into contact with fluid during use are made from a group of first materials to fulfill the high safety and regulatory requirements for materials in contact with pharmaceutical products and intermediates, and any structural components that do not come into contact with fluid during use are made from a group of second materials which are different from the first materials. The first materials can be certified with respect to at least one of the following criteria: bio-safety, chemical compatibility and robustness, extractables and/or leachables, TSE/BSE regulations; whereas the second materials are selected from at least one of the following: recycled plastic, renewable materials. This distinction of materials allows to use highly regulated materials only where needed and therefore to reduce the CO₂ footprint and to save resources and costs.

Moreover, according to a special embodiment of the invention, the distinction of components made from first and second materials allows easy separation and appropriate further processing of the components after use. The fluid-contacting components can be separated from the rest of the grid-module after use without opening the fluid-contacting components so that any residual fluid does not come into contact with the rest of the grid-module. This allows a separation into contaminated / non-contaminated components, a separation into different types of unmixed/homogeneous plastics (recycling), or separation into a recycling portion (reusable returnable packaging) and a disposable portion.

The invention also provides a computer program including an algorithm for creating a digital simulation of a bioprocess device assembly configured by a user based on the modular system according to any of the preceding claims. The computer program allows a user to create a digital version of a bioprocess device assembly - based on a drag & drop functionality of the program, for example - before it is built in the real world. The computer program assists the user in setting up the configuration, and the functionality of the configuration can be tested and simulated, so that costly errors in the real configuration can be avoided.

To assist the user in creating a fully functional bioprocess device assembly, the computer program can include templates for standard configurations, a trouble solver algorithm and an optimization proposal algorithm which could be based on artificial intelligence.

Moreover, the digital version created by the computer program can work as a digital twin of the real bioprocess device assembly for controlling the process performed by the device assembly (especially controlling the pumps, valves etc.) and/or for detection (via the sensors etc.) and correction (adjusting process parameters etc.) of deviations.

Further features and advantages of the invention will become apparent from the following description and from the accompanying drawings to which reference is made. In the drawings:
- Figure 1 shows a top view of a skid for a modular system according to the invention with a plurality of plug-in fields arranged in a two-dimensional grid of a first kind;
- Figure 2 shows a top view of three plug-in structures of the skid;
- Figure 3 shows a top view of a skid with a plurality of plug-in fields arranged in a two-dimensional grid of a second kind;
- Figure 4 shows a perspective bottom view of a grid-module;
- Figure 5 shows a top view of three connected grid-modules on a skid according to a first connection concept;
- Figure 6 shows a lateral view of the three grid-modules of Figure 4;
- Figure 7 shows a universal flow connector for the first connection concept with one universal connector connecting two grid-modules;
- Figure 8 shows a top view of two connected grid-modules according to a second connection concept with two universal flow connectors connecting two grid-modules;
- Figure 9 shows a perspective view of a grid-module with two different universal flow connectors according to the first and second connection concepts, respectively;
- Figure 10 shows a top view of a preassembled group of grid-modules held together in an installation box; and
- Figure 11 shows a top view of three connected grid-modules including sensor inserts; and
- Figure 12 shows a computer running a program for creating a digital simulation of a bioprocess device assembly.

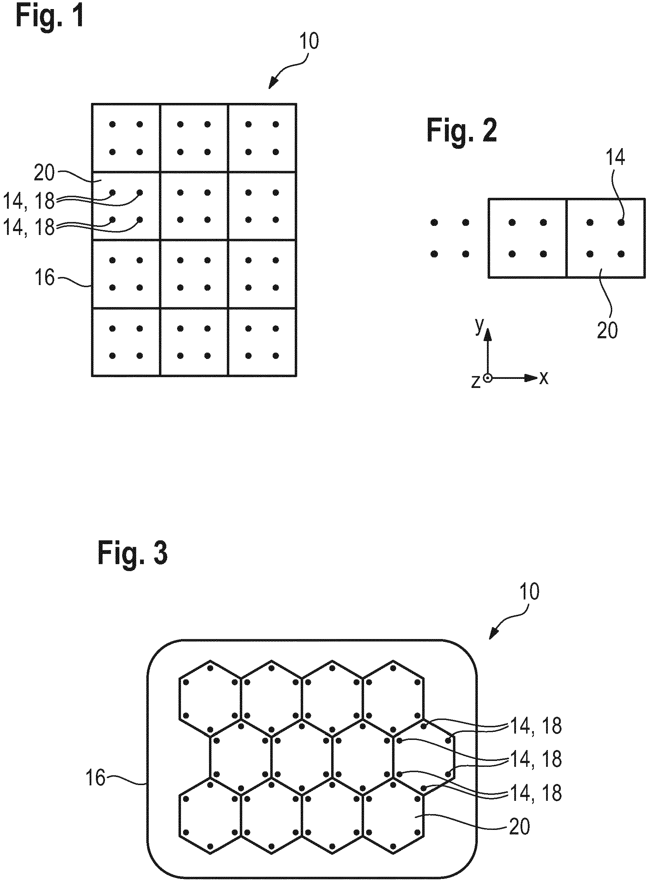

Figure 1 schematically shows a rigid skid 10 for a modular system on which a plurality of grid-modules 12 can be fixed to create an individually configured bioprocess device assembly. The skid 10 includes a plurality of identical plug-in structures 14 provided on a generally flat surface 16. Here, each plug-in structure 14 consists of four mounting holes 18 positioned on the corners of a virtual square. However, the plug-in structures 14 can include any means for a detachable fixation of grid-modules 12 on the skid 10, like screws, threads, magnets, hooks, latches etc.

The plug-in structures 14 are arranged in a regular two-dimensional grid spanning in orthogonal x and y directions. Due to the regular arrangement of the plug-in structures 14, a plurality of two-dimensional plug-in fields 20 of an identical standard shape and an identical standard size are defined, as can be derived from Figure 2.

The two-dimensional grid according to the embodiment shown in Figures 1 and 2 is a rectilinear cartesian grid. This means that the plug-in fields 20 of the skid 10 represent a tessellation by rectangles, in particular squares, and each plug-in field 20 can be addressed by an index (x, y) in two dimensions.

Figure 3 shows another embodiment of a skid 10 with plug-in structures 14 defining another kind of a regular two-dimensional grid. Here, each plug-in structure 14 consists of six mounting holes 18 positioned on the vertices of a virtual regular hexagon. Accordingly, the plug-in fields 20 form a honeycomb pattern.

Irrespective of the actual type of the plug-in fields 20 (rectangles, squares, hexagons, triangles etc.), all plug-in fields 20 of the skid 10 have to be identical with respect to shape and size, and the shape of all plug-in fields 20 has to be that of a regular polygon.

The plug-in fields 20 and/or their boundaries can be marked on the skid 10. Moreover, some of the plug-in fields 20 can include a marking related to a flow direction or the type or function of a grid-module 12 which is to be fixed in the respective plug-in field 20 according to a pre-established configuration scheme (establishment of configuration schemes will be explained later). The markings can be provided in any suitable manner, e.g. by (color) printings, etched structures, illumination, projection, augmented reality (AR) etc.

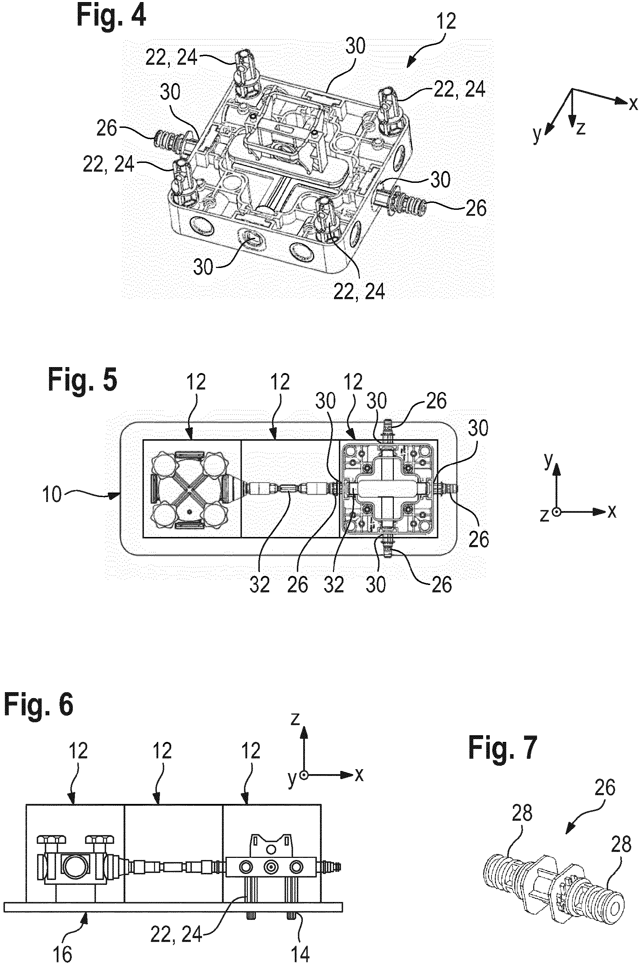

Figure 4 shows a bottom view of an exemplary grid-module 12 taken from a plurality of grid-modules 12 of different types. The grid-module 12 has a counterpart plug-in structure 22 matching any of the plug-in structures 14 of the skid 10. Here, the counterpart plug-in structure 22 includes four mounting studs 24 positioned on the corners of a virtual square, similar to the arrangement of the mounting holes 18 in the plug-in fields 20. The plug-in and counterpart plug-in structures 14, 22 are designed such that the grid-module 12 can be securely fixed in any of the plug-in fields 20 of the skid 10. The actual fixing of the plug-in and counterpart plug-in structures 14, 22 can be based on clip connections, latching connections, clamping connections or the like, e.g. including screws, magnets, hooks, etc.). It is to be noted that the secure fixation is not permanent but separable, i.e. the fixed grid-modules 12 can be detached from the skid 10 if desired, especially after use.

It is also possible that some of the grid-module 12 are provided with plug-in and counterpart plug-in structures at one or more of their lateral sides so that neighboring grid-modules 12 can be fixed to each other (in the plane of the grid). For example, in the grid-module 12 shown in Figure 3 mounting holes are provided on both sides of the connection ports 30 at each lateral side of the grid-module 12. These mounting holes could receive matching mounting studs provided on an opposing lateral side of a neighboring grid-module 12.

In an installed state when fixed to a plug-in structure 14 of the skid 10 and/or to a neighboring grid-module 12, the grid-module 12 has a two-dimensional extension in the plane of the grid that is not greater than the standard size of the plug-in fields 20. This applies to most of the grid-modules 12 that are employed to create a bioprocess device assembly. However, some grid-modules 12 may be larger in x direction and/or y direction. The dimensions of such larger grid-modules 12 in x direction and/or y direction are an integer multiple of the corresponding dimension of a standard size plug-in field 20.

As already indicated, a plurality of grid-modules 12 of different types can be placed and fixed on the skid 10. The different types of grid-modules 12 can be grouped, for example, as follows: Grid-modules 12 having means for actively changing flow properties of the medium, such as valves or pumps, are called flow control grid-modules. Grid-modules 12 having means for sensing, detecting or measuring a property of the medium, such as sensors or probe heads, are called interaction grid-modules. Grid-modules 12 providing a straight or angled fluid path for connecting a remote grid-module 12, and grid-modules 12 including a manifold (e.g. T-piece, Y-piece, crossing) for distributing fluid to several grid-modules 12 or for collecting fluid from several grid-modules 12, are called connector grid-modules and manifold grid-modules, respectively.

Grid-modules 12 may provide more than one function; e.g. a flow control grid-module including a valve can also include a sensor. In such cases the respective grid-module 12 can be assigned to the group related to its primary function (here: flow control).

Although they are the most important, the above-mentioned groups are not exhaustive. Even grid-modules 12 having no fluid-related but other functions can be employed for creating a bioprocess device assembly.

However, the connection and flow communication between the grid-modules 12 - as shown in Figures 5, 6 and 8 by way of example - allowing fluid to flow from one grid-module 12 to another in a defined manner is of major importance for the modular concept. In the following, the distinctive standardized flow connections between the grid-modules 12 will be described.

According to a first connection concept, fluid connections between neighboring grid-modules 12 are established via a common rigid universal flow connector 26 of a first kind which is shown alone in Figure 7. The universal flow connector 26 is symmetrical and has two identical connecting ends 28. The grid-modules 12 include at least one connection port 30 which is in flow communication with at least one integrated fluid line 32 through which a medium to be processed or analyzed can flow. The integrated fluid line 32 can also be a dead-end. In any event, the connection port 30 is adapted to receive one connecting end 28 of such a symmetrical universal flow connector 26. Each connection port 30 of a grid-module 12 is located and designed such that when one of the connecting ends 28 of the universal flow connector 26 is received therein, the opposite free connecting end 28 is necessarily in a standard position and orientation (relative to the plane of the grid) allowing it to be received by an opposing connection port 30 of a neighboring grid-module 12, i.e. a grid-module 12 that is fixed to an adjacent plug-in structure 14.

A plurality of such universal flow connectors 26, all having the same standard shape and the same standard size, is used to provide fluid connections between neighboring grid-modules 12.

In the example shown in Figures 5 and 6, the grid-module 12 on the right has four connection ports 30 for receiving four universal flow connectors 26 having an angular distance of 90°. The connection ports 30 are oriented to allow flow in four different directions parallel to the x and y directions.

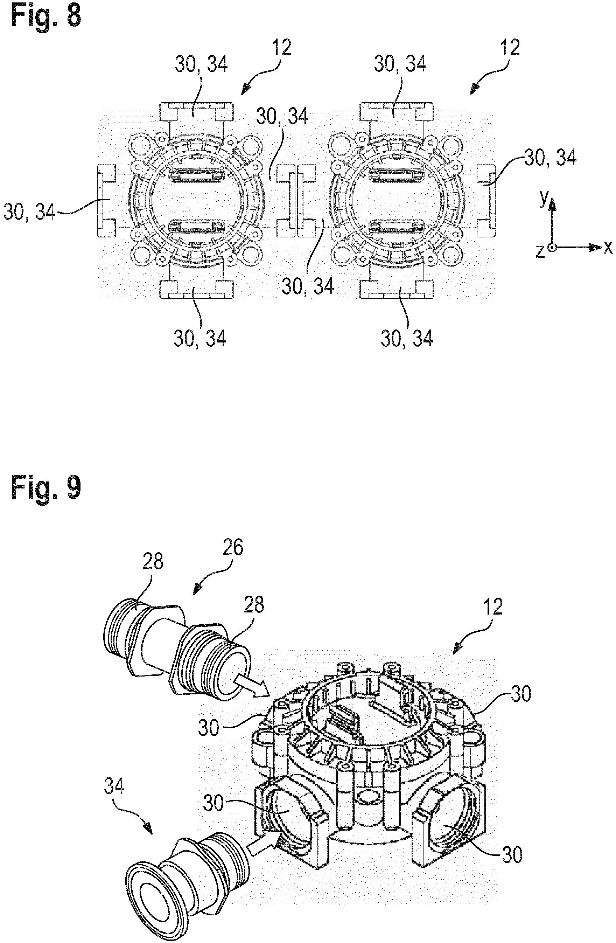

According to a second connection concept, which is indicated in Figure 8, each connection port 30 of a grid-module 12 has its own universal flow connector 34 of a second kind. In this case, the universal flow connector 34 is designed such that it cooperates with the universal flow connector 34 provided in the opposing connection port 30 of the neighboring grid-module 12 to establish a flow connection between the two grid-modules 12. The cooperation can be supported by an additional mechanical connection, such as a tri-clamp connection as shown in Figure 8. Similar to the first concept, all universal flow connectors 34 used in accordance with this connection concept have the same standard shape and the same standard diameter.

Figure 9 illustrates both connection concepts with one grid-module. A universal flow connector 26 of the first kind and a universal flow connector 34 of the second kind are shown, which both can be received in a connection port 30 of the grid-module 12. As already indicated, this includes solutions in which the universal flow connector 26 or 34 is formed in one piece with the connection port 30 (not shown in Figure 9).

The universal flow connector 26 of the first kind connects two neighboring grid-modules 12 with its two connecting ends 28 being received in two opposing connection ports 30 of the neighboring grid-modules 12. In the alternative, two universal flow connectors 34 of the second kind are required to connect two neighboring grid-modules 12. The two universal flow connectors 34 are received in opposing connection ports 30 of the neighboring grid-modules 12, respectively, and their free ends cooperate with each other to establish a flow connection. The connection can be supported by a tri-clamp member or the like, ensuring a tight connection between the free ends.

Both connection concepts in conjunction with the plug-in concept described before allow easy and flexible creation of individual complex tubeless bioprocess device assemblies.

If necessary, any electrical and/or optical connections between neighboring grid-modules 12 can be established in a similarly standardized manner. For such a connection a grid-module 12 includes an electrical and/or optical connector. Such a connector is connected to an electrical and/or optical connector of a neighboring grid-module 12 or to an electrical and/or optical interface of a neighboring grid-module 12. The electrical and/or optical connector can be a universal connector with a standard specification.

Depending on the function of the respective modules, an electrical and/or optical connection between neighboring grid-modules 12 can be provided in addition, or instead of, a flow connection.

If necessary, the skid 10 includes one or more special mounting structures (not shown) at given locations on the skid 10 to attach special elements which need additional components to be connected thereto. For example, at such a given location a pump drive or a valve drive or an optical connector is mounted on the back of the skid 10, so that, for example, a pump head requiring a pump drive, or a valve requiring a valve drive or an optical sensor requiring an optical connector can be fitted to their counterparts. Special mounting structures on the front of the skid 10 can also be used to attach special elements or grid-modules 12 which cannot be fixed to the skid 10 (only) by the regular plug-in structures 14 due to their large size or large weight or due to other reasons.

Figures 5 and 6 show three exemplary grid-modules 12 which are fixed in different manners. The grid-module 12 on the right is fixed to the skid 10 via the plug-in and counterpart plug-in structures 14, 22. The grid-module 12 in the middle, although being placed above a defined plug-in field 20 of the skid 10, is only fixed to its neighboring grid-modules 12 to the left and to the right. The grid-module 12 on the left is placed at a given location as described above. In particular, this grid-module contains a single-use valve which is connected to a valve drive (not shown) mounted on the back of the skid 10. Fixation of the grid-module 12 by plug-in and counterpart plug-in structures 14, 22 may be provided in addition.

Some of the grid-modules 12 have a further plug-in structure 14 at their top side, opposite the counterpart plug-in structure 22. The additional plug-in structure 14 is similar to the plug-in structures 14 of the skid 10, thus allowing another grid-module 12 to be stacked on top. Flow connections and/or electrical connections and/or optical connections between stacked grid-modules 12 can be established as described before, but in z direction.

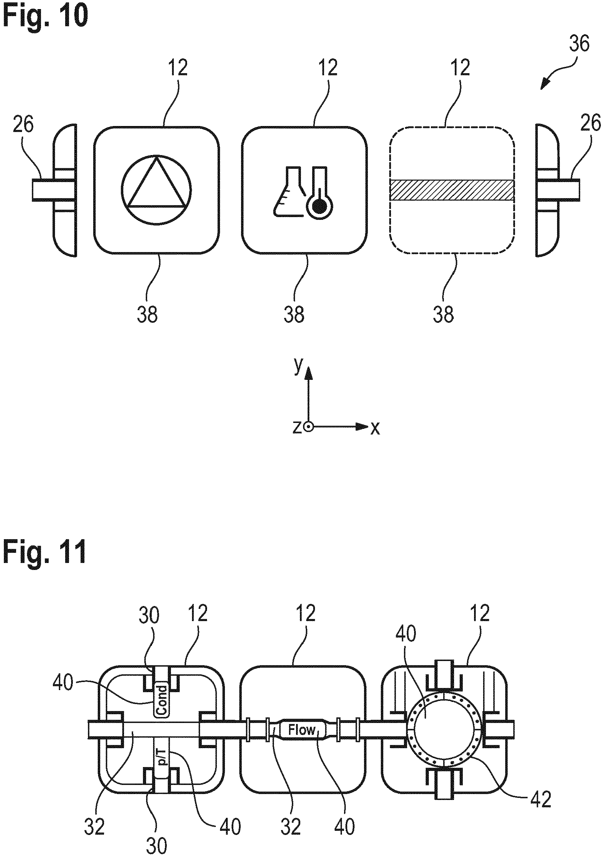

Figure 10 shows a group of grid-modules 12 which are preassembled before they are fixed to the skid 10. The preassembled grid-modules 12 are held together in an installation box 36 or in an installation frame (not shown), thus forming a structural unit. The installation box 36 or installation frame itself can include flow and/or electrical and/or optical connectors as required.

The installation box 36 has an outer surface showing schematic graphical representations 38 of the functions associated with the respective grid-modules 12. Of course, if not installed in a box 36, a grid-module 12 itself can have an outer surface showing a schematic graphical representation 38 of its function.

The installation box 36 itself can be regarded as a skid when it has regularly arranged plug-in structures matching counterpart plug-in structures of the grid-modules 12.

Figure 11 illustrates how functional components 40 like sensors or optical flow cells can be integrated in the different types of grid-modules 12.

The grid-module 12 on the left is an interaction grid-module with four connection ports 30. An integrated fluid line 32 extends between two opposite connection ports 30. The other two connection ports 30 serve as universal receptacles for receiving universal functional components 40. "Universal" here means that the shape and size of all connection ports 30 are identical, and the shapes and sizes of the functional components 40 are adapted to match those of the connection ports 30 so that the functional components 40 can be securely held in the connection ports 30. In the example shown in Figure 11 the functional components 40 are a combined pressure and temperature sensor and a conductivity sensor, both having access to the fluid in the integrated fluid line 32.

Sealing means prevent fluid from leaking through the connection ports 30 where the functional components 40 are received. Of course, the number and location of the functional components 40 in the interaction grid-module can vary.

According to a variant, a universal adapter matching the shape and size (especially the diameter) of the connection ports 30 is used to hold the functional component 40, and, in turn, the adapter together with the functional component 40 is held in a connection port 30.

The grid-module 12 in the middle of Figure 11 is a connector grid-module with an integrated fluid line 32. A functional component 40, e.g. a flow sensor, is integrated or inserted in the fluid line 32. Of, course more than one functional component 40 can be provided in the grid-module 12, especially in case of a manifold grid-module.

The grid-module 12 on the right is a flow control grid-module with a valve block 42. On top of the valve block 42 a functional component 40, here a sensor insert, is arranged. The sensor insert can include an optical flow cell in a bypass line, for example. A probe head can be used in conjunction with the optical flow cell to perform spectroscopic measurements.

Generally, various types of functional components 40, especially sensors, can be integrated in the grid-modules 12. Examples include sensors measuring pressure, differential pressure, pH, conductivity, temperature, refractive index, or optical sensors measuring absorbance or transmittance, optical density, or spectroscopic sensors like NIR, MIR, UV-Vis, fluorescence, or optical scattering sensors such as Raman scattering, multiangle light scattering (MALS) or dynamic light scattering (DLS). Moreover, a sampling port can also be provided, or one of the connection ports 30 or universal flow connectors 26, 34 can be used as a sampling port for aseptic sampling.

Preferably, the functional components 40 are separate units that can be replaced with others or relocated to other grid-modules 12. The functional components 40 can be qualified and tested separately from the grid-modules 12.

As already mentioned, electrical and/or optical connections between neighboring grid-modules 12 can be established via electrical and/or optical connectors and/or interfaces. These electrical and/or optical connections can be used to transfer power to the grid-modules 12 and/or to transfer control signals and/or measurement signals to and/or from the grid-modules 12.

According to a variant, one or more grid-modules 12 can include a wireless communication module for data transfer.

According to another variant, wireless transfer of electric power to a certain grid-module 12 can be realized by providing a power transmitter below the corresponding plug-in field 20. The grid-module 12 includes a power receiver, so that electric power can be transmitted via induction to the grid-module 12 "in z di rection".

Preferably, the whole grid-modules 12 are single-use grid-modules 12 made from materials whose properties are not significantly deteriorated when sterilized by ionizing radiation or by chemicals or by steam. If this is not possible, then the components of the grid-modules 12 which are not single-use components are detachable, so that all single-use components can be discarded together after the other components have been detached.

According to another aspect, any components that come into contact with fluid during use of the bioprocess device assembly are made from materials being certified with respect to criteria related to bio-safety and/or chemical compatibility and robustness and/or extractables and(or leachables and/or TSE/BSE regulations. The materials of the other components can be recycled plastic or renewable materials.

It is to be noted that a grid-module assembly on a skid 10 can be connected to another grid-module assembly on another skid 10, either by one of the connection concepts described above or via additional sterile connectors or in combination with tube lines.

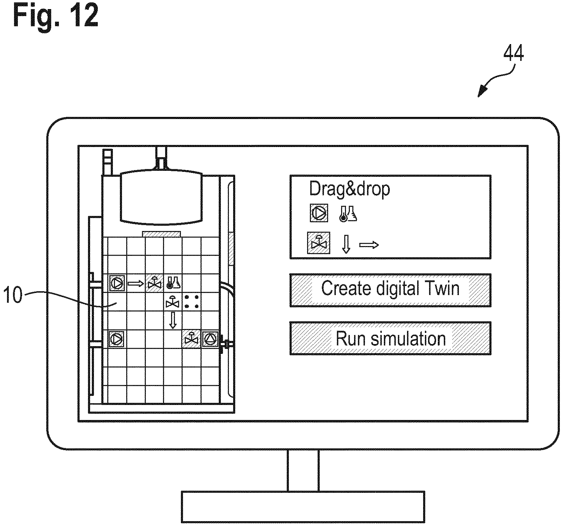

Figure 12 symbolically shows a computer 44 running a program for creating a digital simulation of a bioprocess device assembly. The program is intended to simulate the configuration and the overall functionality of a bioprocess device assembly that can later be created in practice with the modular system including the skid 10 and grid-modules 12 described above. The configuration scheme (layout) of the bioprocess device assembly is directly visualized by the computer program and can be virtually tested before ordering and building a corresponding real bioprocess device assembly. The computer program includes templates for standard configurations, a trouble solver algorithm and an optimization proposal algorithm. In another application the configured bioprocess device assembly simulation can be used as a digital twin e.g. for process control and/or deviation detection and correction.

### List of Reference Signs

- 10: skid
- 12: grid-module
- 14: plug-in structure
- 16: surface
- 18: mounting hole
- 20: plug-in field
- 22: counterpart plug-in structure
- 24: mounting stud
- 26: universal flow connector (first kind)
- 28: connecting end
- 30: connection port
- 32: fluid line
- 34: universal flow connector (second kind)
- 36: installation box
- 38: graphical representation
- 40: functional component
- 42: valve block
- 44: computer

## Claims

1. A modular system for providing a bioprocess device assembly, the modular system comprising:
- a rigid skid (10), and
- a plurality of grid-modules (12);
the skid (10) including a plurality of identical plug-in structures (14), the plurality of plug-in structures (14) being arranged in a regular two-dimensional grid, the grid arrangement of the plug-in structures (14) defining two-dimensional plug-in fields of an identical standard shape and size,
at least some of the grid-modules (12) having a matching counterpart plug-in structure (22) adapted to fix the respective grid-module (12) to any of the plug-in structures (14) of the skid (10),
at least some of the grid-modules (12), in an installed state when fixed to a plug-in structure (14) of the skid (10) and/or to a neighboring grid-module (12), having a two-dimensional extension in the plane of the grid that is not greater than the standard size of the plug-in fields (20),
at least some of the grid-modules (12) including at least one connection port (30) adapted to receive a rigid universal flow connector (26; 34) of standard shape and size in a standard position and orientation,
the connection port (30) of each grid-module (12) defining the standard position and orientation of the universal flow connector (26; 34) such that, in its installed state, the grid-module (12) can be connected via the universal flow connector (26; 34) either directly to the connection port (30) or to another universal flow connector (34) received in the connection port (30) of an opposing grid-module (12) fixed to the grid-module (12) and/or to a neighboring plug-in structure (14),
at least some of the grid-modules (12) having at least one integrated fluid line (32) through, or into, which a medium to be processed or analyzed can flow, the integrated fluid line (32) being in flow communication with the connection port (30) of the grid-module (12);
the plurality of grid-modules (12) including:
- at least one flow control grid-module having means for actively changing flow properties of the medium, and/or
- at least one interaction grid-module having means for sensing, detecting or measuring a property of the medium.

2. The modular system according to claim 1, **characterized in that** the plurality of grid-modules (12) further includes at least one connector grid-module or manifold grid-module.

3. The modular system according to claim 1 or 2, **characterized in that** the two-dimensional extensions of at least some of the grid-modules (12) are identical.

4. The modular system according to any of the preceding claims, **characterized in that** at least one of the grid-modules (12) include four universal flow connectors (26; 34) in a right-angle arrangement.

5. The modular system according to any of the preceding claims, **characterized in that** at least one of the grid-modules (12) includes a further plug-in structure (14) allowing another grid-module (12) to be connected in a direction perpendicular to the plane of the grid via its counterpart plug-in structure (22).

6. The modular system according to any of the preceding claims, **characterized in that** at least one grid-module (12) includes a universal flow connector (26; 34) that, in the installed state of the grid-module (12), is oriented in a direction perpendicular to the plane of the grid.

7. The modular system according to any of the preceding claims, **characterized in that** at least some of the grid-modules (12) include at least one electrical and/or optical connector adapted to be connected to an electrical and/or optical connector of a neighboring grid-module (12) or to an electrical and/or optical interface of a neighboring grid-module (12).

8. The modular system according to any of the preceding claims, **characterized in that** the modular system further comprises at least one non-fluid grid-module without any fluid connection to a neighboring grid-module, but having at least one mechanical and/or electrical and/or optical connector adapted to be connected to a mechanical and/or electrical and/or optical connector of a neighboring grid-module.

9. The modular system according to any of the preceding claims, **characterized in that** a group of grid-modules (12) is preassembled and forms a structural unit before being fixed to the skid (10).

10. The modular system according to claim 9, **characterized in that** the preassembled group of grid-modules (12) is held together in an installation box (36) or by an installation frame, the box (36) or frame optionally including flow and/or electrical and/or optical connectors.

11. The modular system according to any of the preceding claims, **characterized in that** at least some of the grid-modules (12) have an outer surface showing a schematic graphical representation (38) of a function associated to the respective grid-module (12).

12. The modular system according to any of the preceding claims, **characterized in that** at least one of the grid-modules (12) includes a wireless communication module and/or a wireless power receiver.

13. The modular system according to any of the preceding claims, **characterized in that** at least one of the grid-modules (12) includes a universal receptacle for receiving a universal functional component (40).

14. The modular system according to claim 13, **characterized in that** the universal receptacle is integrated into a fluid line (32) or into a flow connector of the grid-module (12).

15. The modular system according to any of the preceding claims, **characterized in that** at least one of the grid-modules (12) includes several functional components (40).

16. The modular system according to any of the preceding claims, **characterized in that** the grid-modules (12) are single-use grid-modules (12) made from materials whose properties are not significantly deteriorated when sterilized by ionizing radiation or by chemicals or by steam.

17. The modular system according to any of the preceding claims, **characterized in that** any components that come into contact with fluid during use are made from a group of first materials and any structural components that do not come into contact with fluid during use are made from a group of second materials which are different from the first materials, the first materials being certified with respect to at least one of the following criteria: bio-safety, chemical compatibility and robustness, extractables and/or leachables, TSE/BSE regulations; whereas the second materials are selected from at least one of the following: recycled plastic, renewable materials.

18. A computer program including an algorithm for creating a digital simulation of a bioprocess device assembly configured by a user based on the modular system according to any of the preceding claims.

19. The computer program according to claim 18, **characterized in that** the computer program includes at least one of the following: templates for standard configurations; a trouble solver algorithm; an optimization proposal algorithm.
